# EUROPÄISCHE PATENTSCHRIFT

(11) **EP 0 528 299 B1**
(45) Veröffentlichungstag und Bekanntmachung des Hinweises auf die Patenterteilung: **11.01.1995**
(21) Anmeldenummer: 92113506.7
(22) Anmeldetag: 07.08.1992
(51) Int. Cl.: C07C 263/20

(54) **Verfahren zur Gewinnung von Polyisocyanaten aus Destillationsrückständen der Toluylendiisocyanatherstellung**
Method of recovery of polyisocyanates from distillation residues of tolueredi-isocyanate preparation
Procédé pour l'extraction de polyisocyanates des résidus de distillation de la préparation de toluèrediisocyanate

(30) Priorität: 20.08.1991 DE 4127514
(43) Veröffentlichungstag der Anmeldung: 24.02.1993
(73) Patentinhaber: BAYER AG, 51368 Leverkusen (DE)
(72) Erfinder: Zarnack, Uwe Jens, Dr., W-2212 Brunsbüttel (DE); Weintritt, Volker, Dr., W-2222 Marne (DE); König, Christian, Dr., W-4044 Kaarst 1 (DE)

(56) Entgegenhaltungen:
- EP-A- 0 017 972
- EP-A- 0 269 218
- FR-A- 2 320 938
- DATABASE WPIL Week 8844, Derwent Publications Ltd., London, GB; AN 88-308139 & DD-A-257 827 (VEB SYNTHESEWERK SCHWARZHEIDE)

## Beschreibung

Die Erfindung betrifft ein neues Verfahren zur Gewinnung von technisch verwertbaren Polyisocyanaten oder Polyisocyanatgemischen aus Destillationsrückständen der Toluylendiisocyanatherstellung.

Bei der großtechnischen Produktion von Toluylendiisocyanat (TDI) durch Phosgenierung von Toluylendiamin entstehen zwangsläufig beträchtliche Mengen an höhermolekularen Folgeprodukten mit Uretdion-, Isocyanurat-, Carbodiimid-, Uretonimin-, Harnstoff- und Biuretstrukturen. Diese Nebenprodukte fallen während der destillativen Auftrennung des rohen Reaktionsgemisches als teerartiger, nicht weiter destillierbarer Rückstand an. Dieser Rückstand belastet einerseits die Wirtschaftlichkeit der industriellen Toluylendiisocyanatherstellung, andererseits sind sie für die üblichen Einsatzzwecke von Polyisocyanaten (Herstellung von Polyurethankunststoffen) ungeeignet.

Ein partielles Recycling dieser großtechnisch unvermeidlich anfallenden TDI-Destillationsrückstände gelang durch alkalische Hydrolyse. Es wird dabei jedoch nur ein kleiner Anteil an Toluylendiaminen während einer außerordentlich langsam ablaufenden Hydrolyse zurückgewonnen (US-PS 3 128 310 oder 3 331 876).

In DE-OS 2 915 830 wird ein Verfahren beschrieben, TDI durch Behandlung des Destillationsrückstandes im Wirbelbett bei 140°C bis 280°C zu gewinnen, wobei nach Abtrennung des Isocyanates der verbleibende Rückstand in staubartiger Konsistenz anfällt. Dieses Verfahren und auch die Verfahren der DD-PS 130 143 oder DE-OS 2 452 804, beschränken sich ausschließlich auf die Gewinnung des im Destillationsrückstand verbliebenen TDI. Allen angeführten Verfahren ist der Nachteil des Anfalles eines nicht weiter verwertbaren Rückstandes gemein.

In US-PS 3 634 361, DE-OS 2 123 183, DE-OS 2 333 150, US-PS 3 634 361 und DE-OS 2 423 594 werden mehrere Verfahren beschrieben, wonach TDI-Destillationsrückstände, die noch bevorzugt über 20 Gew.-% an freien NCO-Gruppen aufweisen, in Anwesenheit von monomeren Diisocyanaten in einem organischen Lösungsmittel, gegebenenfalls unter Anwendung hoher Temperaturen, gelöst werden. Die Anwendbarkeit des Gemisches ist aufgrund seines hohen Lösungsmittelanteils stark eingeschränkt. Weiterhin scheiterte in der Praxis eine solche Verwertung an der mangelnden Lagerstabilität und Standardisierbarkeit der Lösungen sowie an der Sedimentation unlöslicher Bestandteile während der Lagerung.

Desweiteren sind Verfahren zur Verwertung des TDI-Destillationsrückstandes als solche bekannt, wie z.B. der Einsatz des gemahlenen Rückstandes als Füllstoff (DE-OS 2 850 609). Dieses Verfahren bewirkt zwar eine vollständige Nutzung des Destillationsrückstandes, jedoch ist von wesentlichem Nachteil, daß das im Destillationsrückstand enthaltene wertvolle TDI nicht gewonnen werden kann.

Die DD-PS 257 827 beschreibt ein Verfahren, bei welchem der Destillationsrückstand der TDI-Herstellung nach Abmischung mit Diphenylmethandiisocyanat oder dessen höherkernigen Homologen einem destillativen Prozeß unterzogen wird, wobei TDI als Destillat sowie ein Sumpfprodukt als weitere Isocyanatkomponente gewonnen werden. Bei diesem Verfahren erfolgt die Abtrennung des TDI nur unzureichend, was aus toxikologischer und physiologischer Sicht von großem Nachteil ist. Außerdem kann beim Verfahren dieser Vorveröffentlichung entweder der Destillationsrückstand bzw. das zu destillierende Gemisch mit einem Säureakzeptor vorbehandelt oder das anfallende Sumpfprodukt mit einem Säureakzeptor nachbehandelt werden.

Es war die der Erfindung zugrundeliegende Aufgabe, ein neues Verfahren zur Verfügung zu stellen, welches gestattet, TDI-Destillationsrückstände, wie sie bei der technischen Herstellung von TDI anfallen, in eine verwertbare, Isocyanatgruppen aufweisende Komponente zu überführen, und das noch in dem Destillationsrückstand enthaltende, teilweise reversibel chemisch gebundene TDI als weitere Isocyanatkomponente zu isolieren. Der dabei anfallende Destillationssumpf sollte im übrigen, ohne Zusatz von Additiven, wie beispielsweise Säureakzeptoren lagerstabil und weiter verarbeitbar sein, wobei der Restgehalt an TDI in dem Sumpfprodukt unter 200 ppm (Gewicht) liegen sollte.

Diese Aufgabe konnte mit dem nachstehend näher beschriebenen erfindungsgemäßen Verfahren gelöst werden.

Gegenstand der Erfindung ist ein Verfahren zur Gewinnung eines im wesentlichen aus Toluylendiisocyanat bestehenden Destillats und eines Isocyanatgruppen aufweisenden Sumpfprodukts mit einem Gehalt an Toluylendiisocyanat von unter 200 ppm (Gewicht) aus A) Destillationsrückständen der Toluylendiisocyanatherstellung durch Vermischen der Destillationsrückstände A) mit B) gegebenenfalls Urethan- und/oder Allophanat-modifizierten Polyisocyanaten oder Polyisocyanatgemischen der Diphenylmethanreihe mit einem NCO-Gehalt von mindestens 15 Gew.-% und destillative Aufarbeitung des Gemischs, dadurch gekennzeichnet, daß man die Ausgangskomponenten A) und B) über Vorheizer in einen kontinuierlich betriebenen, mit einer Destillationsvorrichtung versehenen Reaktor leitet, das Gemisch vor oder während der destillativen Aufarbeitung auf Temperaturen von 190 bis 250° C erhitzt, einen Druck von 2 bis 100 mbar aufrechterhält und hierdurch eine weitgehende Substitution des im Destillationsrückstand A) reversibel gebundenen Toluylendiisocyanats durch Polyisocyanat B) bewirkt.

Bei den beim erfindungsgemäßen Verfahren einzusetzenden Destillationsrückständen A) handelt es sich um Destillationsrückstände, wie sie bei der Umsetzung von Toluylendiamin mit Phosgen in Gegenwart von Lösungsmitteln und anschließender Destillation der Reaktionslösung anfallen. Unter "Toluylendiamin" (TDA) sind hierbei insbesondere 2,4-Diaminotoluol und seine technischen Gemische mit bis zu 35 Gew.-%, bezogen auf Gemisch, an 2,6-Diaminotoluol zu verstehen. Dementsprechend steht der Begriff "Toluylendiisocyanat" bzw. "TDI" für 2,4-Diisocyanatotoluol oder dessen technischen Gemische mit bis zu 35 Gew.-%, bezogen auf Gemisch, an 2,6-Diisocyanatotoluol. Bei dem beim erfindungsgemäßen Verfahren einzusetzenden TDI-Destillationsrückständen handelt es sich insbesondere um solche, die bei der Herstellung von technischem 2,4-Diisocyanatotoluol, technischen Gemischen aus 80 % 2,4- und 20 % 2,6-Diisocyanatotoluol oder technischen Gemischen aus 65 % 2,4- und 35 % 2,6-Diisocyanatotoluol anfallen. Im allgemeinen enthalten diese technischen Destillationsrückstände bis zu 85 Gew.-% an freiem und reversibel chemisch gebundenem, d.h. thermisch abspaltbarem TDI.

Das erfindungsgemäße Verfahren beruht auf der Beobachtung, daß nicht nur das im Destillationsrückstand gelöste, sondern auch das genannte, reversibel chemisch gebundene TDI unter den Bedingungen des erfindungsgemäßen Verfahrens durch das Polyisocyanat aus B) substituiert werden kann.

Beim erfindungsgemäßen Verfahren wird der Destillationsrückstand A) mit gegebenenfalls Urethan- und/oder Allophanat-modifizierten Polyisocyanaten oder Polyisocyanatgemischen der Diphenylmethanreihe B) vermischt.

Bei den gegebenenfalls Urethan- und/oder Allophanat-modifizierten Polyisocyanaten bzw. Polyisocyanatgemischen der Diphenylmethanreihe handelt es sich um solche mit einem NCO-Gehalt von mindestens 15, vorzugsweise mindestens 25 Gew.-%. Die Komponente B) besteht entweder aus 4,4'-Diisocyanatodiphenylmethan oder dessen technischen Gemischen mit 2,4'- und gegebenenfalls 2,2'-Diisocyanatodiphenylmethan (wobei derartige Gemische vorzugsweise mindestens 50, insbesondere mindestens 70 Gew.-% an 4,4'-Diisocyanatodiphenylmethan enthalten) oder aus Gemischen derartiger Diisocyanatodiphenylmethan-Isomerer mit ihren höheren Homologen mit mehr als zwei Isocyanatgruppen pro Molekül, wobei diese Gemische im allgemeinen bis zu 65 Gew.-% derartiger höherer Homologe enthalten oder schließlich um Urethan- und/oder Allophanatgruppen aufweisenden Semiprepolymere auf Basis dieser Polyisocyanate bzw. Polyisocyanatgemische. Bei diesen Semiprepolymeren handelt es sich um Modifizierungsprodukte der Polyisocyanate oder Polyisocyanatgemische mit unterschüssigen Mengen an mehrwertigen Alkoholen oder um Abmischungen von (i) vorab hergestellten NCO-Prepolymeren oder NCO-Semiprepolymeren auf Basis von Polyisocyanaten oder Polyisocyanatgemischen der genannten Art und bestimmten mehrwertigen Alkoholen mit (ii) unmodifizierten Polyisocyanaten oder Polyisocyanatgemischen der Diphenylmethanreihe. Bei den erfindungsgemäß einsetzbaren Semiprepolymeren handelt es sich vorzugsweise um solche, die zu mindestens 50 Gew.-% aus Urethangruppen-freien Polyisocyanaten der Diphenylmethanreihe bestehen. Als mehrwertige Alkohole zur Urethan- und/oder Allophanat-Modifizierung der Polyisocyanate oder Polyisocyanatgemische sei es in situ durch Zugabe eines Alkohols zu dem Polisocyanat oder Polyisocyanatgemisch oder sei es für eine vorab erfolgende Urethanisierung bzw. Allophanatisierung eines Teils der Polyisocyanate oder Polyisocyanatgemische eignen sich insbesondere Ethergruppen aufweisende, mehrwertige Alkohole des Molekulargewichtsbereichs 106 bis 8 000, vorzugsweise 200 bis 2 000, wie sie durch Alkoxylierung geeigneter, mehrwertiger Startermoleküle unter Verwendung von Ethylenoxid und/oder Propylenoxid in an sich bekannter Weise zugänglich sind. Beispiele sind Diethylenglykol, Dipropylenglykol, Tripropylenglykol, oder Polyetherpolyole auf Basis von Propylenglykol, Glycerin und/oder Trimethylolpropan, die unter Propoxylierung dieser Starter und gegebenenfalls anschließender Ethoxylierung der Propoxylierungsprodukte hergestellt worden sind.

Besonders bevorzugt ist jedoch die Verwendung von Urethan- und Allophanatgruppen-freien Polyisocyanaten oder Polyisocyanatgemischen der Diphenylmethanreihe der oben genannten Art als Komponente B).

Bei der Durchführung des erfindungsgemäßen Verfahrens werden pro 100 Gew.-Teilen TDI-Destllationsrückstand 35 bis 950, vorzugsweise 80 bis 400 Gew.-Teile der Komponente B) verwendet.

Die Gemische werden zur Durchführung des erfindungsgemäßen Verfahrens auf Temperaturen von 190 bis 250°C, vorzugsweise 200 bis 230°C erhitzt und gleichzeitig und/oder im Anschluß an die Hitzebehandlung destillativ aufgearbeitet.

Vorzugsweise erfolgt die destillative Aufarbeitung gleichzeitig mit der Hitzebehandlung unter Verwendung eines kontinuierlich betriebenen Reaktors, der mit einer Destillationsanlage verknüpft ist. Das vorzugsweise einstufig in derartigen Reaktoren durchgeführte erfindungsgemäße Verfahren wird vorzugsweise unter einem Druck von 2 bis 100, vorzugsweise 5 bis 40 mbar durchgeführt.

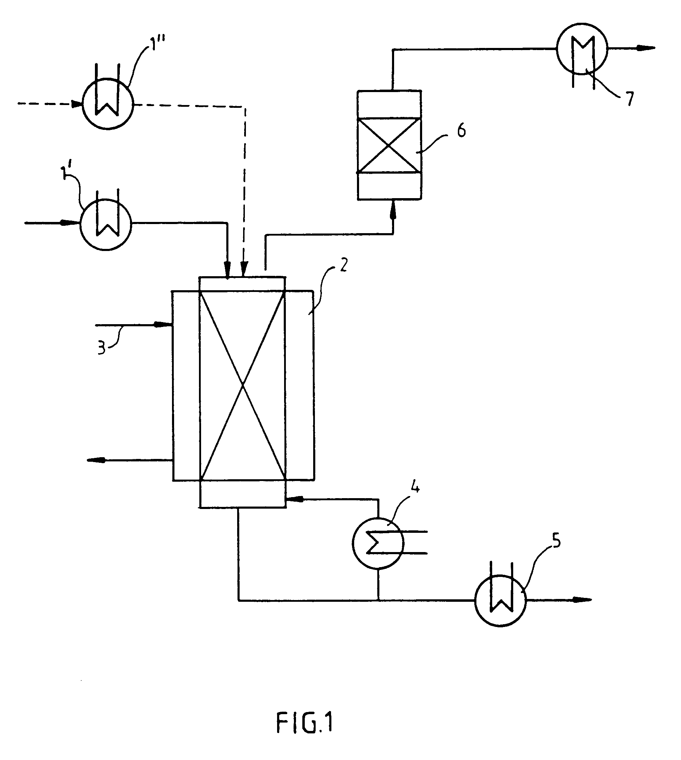

Besonders gut zur Durchführung des erfindungsgemäßen Verfahrens geeignet ist die in nachstehender Fig. 1 dargestellte Vorrichtung. In dieser bedeuten
1') und 1'') einen Vorheizer;
2) den Reaktor;
3) den Wärmeträger;
4) einen Wärmeaustauscher;
5) einen Quenchkühler;
6) eine Destillationskolonne und
7) einen Kondensator.

Besonders bevorzugt werden solche Reaktoren (2) verwendet, in die zwecks Erhöhung der Verweilzeit Glockenboden ähnliche Einbauten und/oder Strukturpackungen, wie sie in der Destillationstechnik üblich sind, eingebaut sind. Die Destillationskolonne (6) wird vorzugsweise unter solchen Temperatur- und Druckbedingungen gefahren, daß als Überkopfprodukt im Kondensator (7) ausschließlich TDI mit einer Reinheit von mindestens 99,5 % anfällt, während praktisch die Gesamtmenge der eingesetzten Komponente B) über den Sumpf entnommen wird. Andererseits werden die Temperatur- und Druckbedingungen im Reaktor (2) und in der Destillationskolonne (6) innerhalb der genannten Bereiche so gewählt, daß das freigesetzte bzw. durch Polyisocyanate B) substituierte TDI praktisch ausschließlich als Destillat anfällt, bzw. daß der TDI-Gehalt im Sumpfprodukt bei maximal 200, vorzugsweise maximal 125 ppm (Gewicht) liegt.

Es hat sich als vorteilhaft erwiesen, die Mengenverhältnisse der Ausgangskomponenten A) und B) innerhalb der genannten Bereiche so zu bemessen, daß der Gehalt des über den Kühler (5) entnommenen Sumpfprodukts an Destillationsrückstandsanteilen bei maximal 30 Gew.-% liegt.

Die Ausgangskomponenten A) und B) können sowohl bereits vor dem Reaktor (2) vermischt werden und als Gemisch, gegebenenfalls über einen der Vorheizer (1') bzw. (1'') oder getrennt, vorzugsweise über die genannten Vorheizer in den Reaktor geleitet werden, so daß erst im Reaktor selbst das Gemisch aus A) und B) entsteht. Im allgemeinen werden die Ausgangskomponenten A) und B) bzw. das aus diesem vorab hergestellte Gemisch in dem Vorheizer bzw. den Vorheizern auf Temperaturen von mindestens 70°C vorerhitzt während die weitere Aufheizung des Gemischs im Reaktor erfolgt.

Gemäß einer bevorzugten Variante des erfindungsgemäßen Verfahrens wird das mit einer Temperatur von mindestens 190°C anfallende Sumpfprodukt während eines Zeitraums von maximal 1 Minute auf unter 80°C, vorzugsweise unter 60°C abgekühlt, in dem es beipielsweise den Quenchkühler (5) durchläuft. Hierdurch wird erreicht, daß die resultierende Lösung ohne weitere Zusätze lager- und viskositätsstabil bleibt.

Das als Sumpfprodukt anfallende, Isocyanatgruppen aufweisende Gemisch weist im allgemeinen bei 25°C eine Viskosität von 200 bis 10 000 mPa.s und einen NCO-Gehalt von 23 bis 31 Gew.-% auf. Wegen seines niedrigen Gehalts an TDI stellt es ein wertvolles Ausgangsmaterial zur Herstellung von Polyurethan-Kunststoffen dar, bei dessen Verarbeitung selbst bei hohen Verarbeitungstemperaturen die TDI-Emissionen weit unter der maximalen Arbeitsplatzkonzentration liegen. Eine nachträgliche TDI-Freisetzung kann auch bei höheren Verarbeitungs- und Einsatztemperaturen ausgeschlossen werden.

Das als Destillat gewonnene TDI kann seinerseits für alle Einsatzgebiete für TDI verwendet werden. Gewünschtenfalls kann es zusammen mit rohem Frisch-TDI einer weiteren destillativen Aufarbeitung zugeführt werden.

Das nachstehende Beispiel dient der weiteren Erläuterung des erfindungsgemäßen Verfahrens.

### Beispiel (Vgl. Zeichnung)

In den bei 200°C und 8 mbar betriebenen Reaktor (2) einer kontinuierlich betriebenen Laboranlage gemäß. Zeichnung wird ein über den Vorheizer (1') auf 90°C erhitztes Gemisch aus 4 424 g/h TDI-Destillationsrückstand mit 5 397 g/h Diisocyanatodiphenylmethan kontinuierlich eingespeist. Bei dem TDI-Destillationsrückstand handelt es sich um einen Destillationsrückstand mit einem Gehalt an gelöstem und thermisch abspaltbarem TDI von insgesamt 66,3 Gew.-%, der bei der destillativen Aufarbeitung des Phosgenierungsprodukts von Toluylendiamin (80 Gew.-% 2,4- und 20 Gew.-% 2,6-Isomere) in Dichlorbenzol anfiel. Bei dem Diisocyanatodiphenylmethan handelte es sich um ein Isomerengemisch aus 93,8 Gew.-% 4,4'-, 5,9 Gew.-% 2,4'- und 0,3 Gew.-% 2,2'-Diisocyanatodiphenylmethan.

Das den Reaktor in einer Menge von 6 823 g/h und mit einer Temperatur von 200°C verlassende Sumpfprodukt wird im Quenchkühler (5) (mittlere Verweilzeit im Quenchkühler: 30 Sekunden auf 50°C abgeschreckt. Das so erhaltene viskositäts- und sedimentationsstabile Produkt weist bei 25°C eine Viskosität von 326 mPa.s, einen Gehalt an TDI von 60 ppm (Gewicht) und einen NCO-Gehalt von 28 Gew.-% auf. Es läßt sich ohne weiteres als Polyisocyanatkomponente zur Herstellung von Polyurethankunststoffen nach dem Isocyanat-Polyadditions-Verfahren einsetzen.

Das über Kopf der Kolonne (6) in einer Menge von 2933 g/h gewonnene TDI (Kopftemperatur der Kolonne: 115°C) weist einen Restgehalt von 0,04 Gew.-% Diisocyanatodiphenylmethan auf. Es kann der für TDI üblichen Verwendung, beispielsweise zur Herstellung von Polyurethan-Weichschaumstoffen zugeführt werden.

## Patentansprüche

1. Verfahren zur Gewinnung eines im wesentlichen aus Toluylendiisocyanat bestehenden Destillats und eines Isocyanatgruppen aufweisenden Sumpfprodukts mit einem Gehalt an Toluylendiisocyanat von unter 200 ppm (Gewicht) aus A) Destillationsrückständen der Toluylendiisocyanatherstellung durch Vermischen der Destillationsrückstände A) mit B) gegebenenfalls Urethan- und/oder Allophanat-modifizierten Polyisocyanaten oder Polyisocyanatgemischen der Diphenylmethanreihe mit einem NCO-Gehalt von mindestens 15 Gew.-% und destillative Aufarbeitung des Gemischs, dadurch gekennzeichnet, daß man die Ausgangskomponenten A) und B) über Vorheizer in einen kontinuierlich betriebenen, mit einer Destillationsvorrichtung versehenen Reaktor leitet, das Gemisch vor oder während der destillativen Aufarbeitung auf Temperaturen von 190 bis 250° C erhitzt, einen Druck von 2 bis 100 mbar aufrechterhält und hierdurch eine weitgehende Substitution des im Destillationsrückstand A) reversibel gebundenen Toluylendiisocyanats durch Polyisocyanat B) bewirkt.

2. Verfahren gemäß Anspruch 1, dadurch gekennzeichnet, daß man pro 100 Gew.-Teile Destillationsrückstands A) 35 bis 950 Gew.-Teile Polyisocyanat oder Polyisocyanatgemisch B) verwendet.

3. Verfahren gemäß Anspruch 1 und 2, dadurch gekennzeichnet, daß man als Komponente B) 4,4'-Diisocyanatodiphenylmethan, seine technischen Gemische mit 2,4'- und gegebenenfalls 2,2'-Diisocyanatodiphenylmethan oder Gemische dieser Diisocyanatodiphenylmethan-Isomeren mit bis zu 65 Gew.-%, bezogen auf Gemisch, ihrer höheren, mehr als 2 Isocyanatgruppen pro Molekül aufweisenden Homologen verwendet.

4. Verfahren gemäß Anspruch 1 bis 3, dadurch gekennzeichnet, daß man das mit einer Temperatur von mindestens 190° C anfallende Sumpfprodukt innerhalb eines Zeitraums von maximal 1 Minute auf eine Temperatur von unter 80° C abschreckt.

## Claims

1. A process for recovering a distillate consisting essentially of tolylene diisocyanate and an isocyanate-group-containing bottom product having a tolylene diisocyanate content of less than 200 ppm (by weight) from A) distillation residues from the production of tolylene diisocyanate by mixing of the distillation residues A) with B) optionally urethane- and/or allophanate-modified polyisocyanates or polyisocyanate mixtures of the diphenyl methane series having an NCO content of at least 15% by weight and working up of the mixture by distillation, characterized in that the starting components A) and B) are passed through preheaters into a continuously operated reactor equipped with a distillation column, the mixture is heated to temperatures of 190 to 250°C before or during working up by distillation and a pressure of 2 to 100 mbar is maintained so that the tolylene diisocyanate reversibly bound in the distillation residue A) is largely substituted by polyisocyanate B).

2. A process as claimed in claim 1, characterized in that 35 to 950 parts by weight of polyisocyanate or polyisocyanate mixture B) are used per 100 parts by weight distillation residue A).

3. A process as claimed in claims 1 and 2, characterized in that 4,4'-diisocyanatodiphenyl methane, technical mixtures thereof with 2,4'- and, optionally, 2,2'-diisocyanatodiphenyl methane or mixtures of these diisocyanatodiphenyl methane isomers with up to 65% by weight, based on the mixture, of their higher homologs containing more than two isocyanate groups per molecule are used as component B).

4. A process as claimed in claims 1 to 3, characterized in that the bottom product which accumulates with a temperature of at least 190°C is quenched to a temperature below 80°C in at most 1 minute.

## Revendications

1. Procédé de récupération d'un distillat essentiellement constitué de toluylène diisocyanate et d'un produit de queue présentant des groupes isocyanates avec une teneur en toluylène diisocyanate inférieure à 200 ppm (en poids) à partir de A) résidus de distillation provenant de la préparation de toluylène diisocyanate par mélange des résidus de distillation A) avec B) polyisocyanates ou mélanges de polyisocyanates de la série des diphénylméthanes, éventuellement modifiés à l'uréthanne et/ou à l'allophanate, présentant une teneur en NCO d'au moins 15 % en poids et par retraitement du mélange par distillation, caractérisé en ce qu'on introduit les constituants de départ A) et B) par l'intermédiaire d'un préchauffeur dans un réacteur fonctionnant en continu, muni d'un dispositif de distillation, on chauffe le mélange avant ou pendant le retraitement par distillation à des températures de 190 à 250°C, on maintient une pression comprise entre 2 et 100 mbars et on provoque par là une importante substitution du toluylène diisocyanate réversiblement lié dans le résidu de distillation A) par le polyisocyanate B).

2. Procédé selon la revendication 1, caractérisé en ce qu'on utilise pour 100 parties en poids du résidu de distillation A) 35 à 950 parties en poids de polyisocyanate ou de mélange de polyisocyanates B).

3. Procédé selon les revendications 1 et 2, caractérisé en ce qu'on utilise comme constituant B) du 4,4'-diisocyanatodiphénylméthane, ses mélanges industriels comprenant du 2,4'- et éventuellement du 2,2'-diisocyanatodiphénylméthane ou des mélanges de ces isomères du diisocyanatodiphénylméthane comprenant jusqu'à 65 % en poids, rapportés au mélange, de leurs homologues supérieurs présentant plus de deux groupes isocyanates par molécule.

4. Procédé selon les revendications 1 à 3, caractérisé en ce qu'on fait tremper à une température inférieure à 80°C le produit de queue produit à une température d'au moins 190°C en l'espace de maximum 1 min.
